# EUROPEAN PATENT SPECIFICATION

(11) **EP 2 710 142 B1**
(45) Date of publication and mention of the grant of the patent: **26.04.2017**
(21) Application number: 12721311.4
(22) Date of filing: 18.05.2012
(51) Int. Cl.: C12Q 1/68, G01N 33/574

(54) **SIGNATURE FOR THE DIAGNOSIS OF CANCER AGGRESSIVENESS AND GENETIC INSTABILITY**
SIGNATUR FÜR DIE DIAGNOSE DER KREBSAGGRESSIVITÄT UND GENETISCHER INSTABILITÄT
SIGNATURE POUR LE DIAGNOSTIC DE L'AGRESSIVITÉ ET L'INSTABILITÉ GÉNÉTIQUE DU CANCER

(30) Priority: 18.05.2011 EP 11305603; 18.05.2011 US 201113110749
(43) Date of publication of application: 26.03.2014
(73) Proprietor: Centre National de la Recherche Scientifique (C.N.R.S.), 75016 Paris (FR)
(72) Inventor: CAZAUX, Christophe, F-31830 Plaisance Du Touch (FR); HOFFMANN, Jean-Sebastien, F-31500 Toulouse (FR)
(74) Representative: Regimbeau
(86) International application number: PCT/EP2012/059239
(87) International publication number: WO 2012/156501

(56) References cited:
- EP-A1- 2 309 002
- EP-A1- 2 322 658
- WO-A2-2011/058367
- US-A1- 2010 273 711
- KAWAMURA KIYOKO ET AL: "DNA polymerase theta is preferentially expressed in lymphoid tissues and upregulated in human cancers", INTERNATIONAL JOURNAL OF CANCER, JOHN WILEY & SONS, INC, UNITED STATES, SWITZERLAND, GERMANY, vol. 109, no. 1, 10 March 2004 (2004-03-10), pages 9-16, XP002567045, ISSN: 0020-7136, DOI: 10.1002/IJC.11666 [retrieved on 2003-12-04] cited in the application
- F. LEMEE ET AL: "DNA polymerase up-regulation is associated with poor survival in breast cancer, perturbs DNA replication, and promotes genetic instability", PROCEEDINGS OF THE NATIONAL ACADEMY OF SCIENCES, vol. 107, no. 30, 27 July 2010 (2010-07-27), pages 13390-13395, XP055008221, ISSN: 0027-8424, DOI: 10.1073/pnas.0910759107 cited in the application
- DANUTA GALETZKA ET AL: "Expression of somatic DNA repair genes in human testes", JOURNAL OF CELLULAR BIOCHEMISTRY, vol. 100, no. 5, 1 April 2007 (2007-04-01) , pages 1232-1239, XP055008222, ISSN: 0730-2312, DOI: 10.1002/jcb.21113
- Fanny Lemée: "Dérégulation de l'expression des ADN polymérases translésionnelles, instabilité génétique et progression des cancers colorectaux et du sein", Thèse de doctorat, université de Toulouse , 12 June 2009 (2009-06-12), XP055008256, Retrieved from the Internet: URL:http://thesesups.ups-tlse.fr/604/1/Lem ee_Fanny.pdf [retrieved on 2011-09-28]
- HOFFMANN J S ET AL: "Aberrant expression of alternative DNA polymerases: A source of mutator phenotype as well as replicative stress in cancer", SEMINARS IN CANCER BIOLOGY, SAUNDERS SCIENTIFIC PUBLICATIONS, PHILADELPHIA, PA, US, vol. 20, no. 5, 1 October 2010 (2010-10-01), pages 312-319, XP027525219, ISSN: 1044-579X [retrieved on 2010-10-01]
- Anonymous: "TaqMan Low Density Array Formats", Applied Biosystems , 2007, XP055008225, Retrieved from the Internet: URL:http://www3.appliedbiosystems.com/cms/ groups/mcb_marketing/documents/generaldocu ments/cms_040413.pdf [retrieved on 2011-09-27]
- MATAKIDOU A ET AL: "GENETIC VARIATION IN THE DNA REPAIR GENES IS PREDICTIVE OF OUTCOME IN LUNG CANCER", 20070101, vol. 16, no. 19, 1 January 2007 (2007-01-01), pages 2333-2340, XP003024922, DOI: 10.1093/HMG/DDM190
- G. S. HIGGINS ET AL: "A Small Interfering RNA Screen of Genes Involved in DNA Repair Identifies Tumor-Specific Radiosensitization by POLQ Knockdown", CANCER RESEARCH, vol. 70, no. 7, 1 April 2010 (2010-04-01), pages 2984-2993, XP055008233, ISSN: 0008-5472, DOI: 10.1158/0008-5472.CAN-09-4040
- VANDESOMPELE JO ET AL: "Accurate normalization of real-time quantitative RT-PCR data by geometric averaging of multiple internal control genes", GENOME BIOLOGY, BIOMED CENTRAL LTD., LONDON, GB, vol. 3, no. 7, 18 June 2002 (2002-06-18), XP021021149, ISSN: 1465-6906, DOI: 10.1186/GB-2002-3-7-RESEARCH0034
- ANONYMOUS: 'PCR applications manual, 3rd edition', [Online] 01 January 2006, XP055034540 Retrieved from the Internet: <URL:https://www.roche-applied-science.com/ publications/print_mat/pcr_application_manu al_3rd_edition.pdf> [retrieved on 2012-08-02]
- MENGUAL LOURDES ET AL: 'Multiplex preamplification of specific cDNA targets prior to gene expression analysis by TaqMan Arrays' BMC RESEARCH NOTES, BIOMED CENTRAL LTD, GB vol. 1, no. 1, 21, 05 June 2008, pages 1 - 8, XP021045871 DOI: 10.1186/1756-0500-1-21 ISSN: 1756-0500
- CHEN G ET AL: "Discordant protein and mRNA expression in lung adenocarcinomas", MOLECULAR & CELLULAR PROTEOMICS, AMERICAN SOCIETY FOR BIOCHEMISTRY AND MOLECULAR BIOLOGY, vol. 1, no. 4, 1 April 2002 (2002-04-01), pages 304-313, XP008123587, ISSN: 1535-9476, DOI: 10.1074/MCP.M200008-MCP200 [retrieved on 2002-03-12]
- SUZANNE K LAU ET AL: "Three-gene prognostic classifier for early-stage non-small-cell lung cancer", JOURNAL OF CLINICAL ONCOLOGY, AMERICAN SOCIETY OF CLINICAL ONCOLOGY, US, vol. 25, no. 35, 10 December 2007 (2007-12-10), pages 5562-5569, XP008145645, ISSN: 0732-183X, DOI: 10.1200/JCO.2007.12.0352

## Description

### FIELD OF THE INVENTION

The present invention is in the field of cancer management, including diagnosis of aggressiveness and genetic instability of said cancer, and selection of an appropriate treatment. The invention is based on the finding that overexpression of POLQ is highly related to aggressiveness of a tumor, and thus to survival of the patient. The same overexpression is also correlated to genetic instability, which may then be used to kill tumor cells. Radiotherapy, for instance, is more efficacious on DNA which is already damaged. Likewise, DNA damage checkpoint or DNA replication licensing/initiation or DNA repair inhibitors may prevent signaling or repair of DNA damage, thus leading to tumor cell death (synthetic sickness lethality).

### BACKGROUND ART

Cancer is a multi-faceted disease in which a group of cells display uncontrolled growth, invasion that intrudes upon and destroys adjacent tissues, and sometimes metastasis, or spreading to other locations in the body via lymph or blood. These three malignant properties of cancers differentiate them from benign tumors, which do not invade or metastasize.

There are a number of methods currently used to treat each type of cancer, including surgery, radiotherapy, and chemotherapy. Successful cancer therapy is directed to the primary tumor and to any metastases, whether clinically apparent or microscopic.

The selection of an appropriate treatment is crucial for the patient. It is essential to know when to use immediately a heavy and aggressive treatment protocol in order to prevent extension of an aggressive cancer. In contrast, performing a heavy and aggressive treatment when it is not necessitated by the tumor carried by the patient is also disadvantageous for the patient. Indeed, heavy and aggressive treatments always lead to adverse toxicities that may significantly affect the patient's quality of life. For example most of them are mutagenic and are thus prone to induce secondary tumors.

In addition, such heavy and aggressive treatments are usually very costly, and should thus be performed only when it is necessary.

Currently, treatment selection for solid tumors is based on tumor staging, which is usually performed using the Tumor/Node/Metastasis (TNM) test from the American Joint Committee on Cancer (AJCC). The TNM system assigns a number based on three categories. "T" denotes the tumor size, "N" the degree of lymphatic node involvement, and "M" the degree of metastasis. The broader stage of a cancer is usually quoted as a number I, II, III, IV derived from the TNM value grouped by prognosis; a higher number indicates a more advanced cancer and likely a worse outcome.

It is commonly acknowledged that, while this test and staging system provides some valuable information concerning the stage at which solid cancer has been diagnosed in the patient, it is imprecise and insufficient. In particular, it fails to identify the earliest stages of tumor progression. In addition, the TNM test does not give information on the tumor aggressiveness and its usefulness for prognosis is thus limited. Finally, it is limited to solid tumors. Liquid tumors on the other hand are mostly characterized by the identification of cytogenetic alterations.

Several protein and genetic markers have been described in an attempt to refine prognostic information. In particular, gene expression analysis has allowed the identification of mutli-gene prognostic signatures. However, the information gathered in different studies has often proven confusing (see e.g. Lenz, Gastrointest Cancer Res, 1(4 Suppl 2): S29-32, 2007; Walther et al., Nat Rev Cancer, 9(7): 489-99, 2009; Sotiriou & Pusztai, New England J Med, 360: 790-800, 2009; Subramanian.& Simon, J Natl Cancer Inst., 102: 464-468, 2010). Overlap between different signatures for the same cancer, for example, can be poor. The robustness of multi-gene signatures is also questionable because they essentially concern cell cycle- or proliferation-associated genes and therefore add nothing to standard clinico-pathological staging. In addition, each signature is limited to a specific type of cancer. None of these markers is thus in routine clinical use.

There is a real need for better prognosis tests of cancer, not only to improve patient global survival, but also to improve their quality of life and to keep aggressive and costly chemotherapies for patients who will really benefit from them. In particular, there is a need for a single-gene prognosis marker which can be used reliably for the prognosis of as many types of cancers as possible.

In normal cell proliferation, DNA replication is performed by 3 DNA polymerases known as replicating polymerases (POLA, POLD and POLE). However, 10 other DNA polymerases have been identified in human cells, which are known as specialized polymerases and which functions are still largely unknown. These specialized polymerases appear to have been maintained through evolution because of their ability to process despite of DNA damage. It also appears that these polymerases are very mutagenic and that their activity is tightly controlled.

POLQ (also known as POL theta or POLθ) is one of these specialized DNA polymerases, and contains a helicase domain in its N-terminal portion and a polymerase domain in its C-terminus. Although the function of this particular specialized DNA polymerase is still largely unknown, it appears to be involved in maintenance of genome stability and in DNA repair (Seki et al., EMBO J, 23: 4484-4494, 2004; Masuda et al., Proc. Natal. Acad. Sci. U.S.A., 102: 13986-13991, 2005, Yoshimura et al., Mol. Cell, 24 : 115-125, 2006), but also in the licensing and initiation of DNA replication, probably by facilitating the firing of the replication origins (unpublished data).

POLQ expression has been analyzed in various tumors (Kawamura et al., Int J Cancer, 109: 9-16, 2004; Pillaire et al., Oncogene, 29(6):876-887, 2010; Lemée et al., Proc Natl Acad Sci U S A., 107(30): 13390-5, 2010; Higgins et al., Oncotarget, 1(3): 175-184, 2010). In particular, Kawamura et al. (Int J Cancer, 109: 9-16, 2004) detected increased levels of expression of POLQ mRNA in a number of different cancerous tissues. However, mRNA levels measurement as performed by Northern blotting, a technique which is nowhere as sensitive as methods such as quantitative PCR. In addition, the number of patients studied was very small and it thus very difficult to draw any meaningful conclusion from this study. For example, the number of patients suffering from adenocarcinomas was just 7. The robustness and the significance of these results therefore seem open to question. The inventors analyzed the variation of expression of the POLQ gene in tumor versus normal tissues and compared these data with disease progression and clinical features. They showed that POL Q was significantly overexpressed in tumor tissues. In addition, they demonstrated that POLQ deregulated expression contributed actively to tumor progression. Indeed, POLQ overexpression leads to genetic instability and notably DNA damage.

### DESCRIPTION OF THE INVENTION

The present inventors have shown that POLQ is overexpressed in various different cancers and this overexpression gives information about the patient prognosis For example, POLQ was found to be overexpressed in most of 93 lung cancers. This overexpression was associated to the patient survival, whatever the survival term examined (overall survival, relapse-free survival, disease-free survival). Remarkably, the statistical link between POLQ and patient survival is independent of the tumor stage and of the treatment.

The present invention thus provides a method for diagnosing the aggressiveness of a cancer in a patient, as defined in claim 1. According to the method of the invention, elevated expression levels of the POLQ gene indicate aggressiveness of said cancer.

Therefore, the present invention provides a method for diagnosing aggressiveness of a cancer in a patient from a cancer sample of said patient, comprising:
a) measuring in vitro the expression level of the POLQ gene and the expression level of a control gene in said patient cancer sample,
b) calculating for said POLQ gene an expression level ratio of the expression level of POLQ to the expression of the said control gene in said patient cancer sample,
c) comparing the said POLQ expression level ratio to a corresponding threshold value, and
d) diagnosing cancer aggressiveness if the said POLQ expression level ratio is superior to its corresponding threshold value,
wherein said cancer is adenocarcinoma of the lung.

In another aspect, the method of the invention allows for the detection of cancer cells displaying genetic instability, i.e. DNA damage or "replicative stress" caused by a perturbation of origin firing. Deregulated expression of POLQ leads to increased mitotic abnormalities, such as chromatid breaks, chromosomal end-to-end fusions, dicentric chromosomes, and other abnormalities. Thus POLQ overexpression leads to DNA damage and chromosome instability.

Therefore, the present invention provides a method for diagnosing genetic instability in a cancer in a patient from a cancer sample of said patient, comprising:
a) measuring in vitro the expression level of the POLQ gene and the expression level of a control gene in said patient cancer sample,
b) calculating for said POLQ gene an expression level ratio of the expression level of POLQ to the expression of the said control gene in said patient cancer sample,
c) comparing the said POLQ expression level ratio to a corresponding threshold value, and
d) diagnosing cancer genetic instability if the said POLQ expression level ratio is superior to its corresponding threshold value,
wherein said cancer is adenocarcinoma of the lung.

It is understood that both methods can be combined. Therefore, in a further aspect, the invention relates to a method for diagnosing the aggressiveness of a cancer in a patient and for the detection of cancer cells displaying genetic instability. The method of the invention thus presents the advantage of allowing the detection of a genetic event associated with both a bad survival prognosis and genetic instability.

As used herein, the terms "cancer" and "cancerous" refer to or describe the physiological condition in mammals that is typically characterized by unregulated cell growth. The terms "cancer" and "cancerous" as used herein are meant to encompass all stages of the disease. Thus, a "cancer" as used herein may include both benign and malignant tumors. Advantageously, the said cancer is an early or mid-stage cancer.

By "early/Mid stage", it is herein meant a cancer whose stage is comprised between stage IA and stage IIIA. One of the earliest events to take place in precancerous cells is "replicative stress" caused by a perturbation of origin firing. The present inventors have shown that POLQ interacts with proteins involved in replication initiation and that its deregulation interferes with origin licensing by perturbing the timing of origins. The inventors show that POLQ is probably more involved in the initiation than elongation of DNA replication. Thus POLQ is a particularly useful marker for cancers in early stages.

In a preferred aspect, the cancer of the method of the disclosure is a cancer in an early stage. Examples of cancer include but are not limited to, carcinoma, lymphoma, blastoma, sarcoma, and leukemia or lymphoid malignancies.

More particular examples of such cancers include, but not limited to, squamous cell cancer (e.g., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AID S -related lymphoma; and Waldenstrom's Macroglobulinemia); chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases.

In a particular embodiment, cancers that can be prognosed using the method of the disclosure include any type of cancer except breast cancer. In another embodiment, the cancers of the disclosure include any type of cancers except colon cancer. In yet another embodiment, all cancers are amenable to the methods of the disclosure except breast cancer and colon cancer. In yet another embodiment, the methods of the disclosure can be used with any combination of 2, 3, 4 or more of the cancers listed above.

In the most preferred embodiment, the method of the disclosure is used with lung cancer.

As used herein, the term "POLQ" refers to the human gene encoding the DNA polymerase theta (Entrez Gene ID number: 10721; mRNA sequence reference: NM_199420.3; protein sequence reference: NP_955452.3). In addition, the invention encompasses all the isoforms of the said POLQ gene. Isoform, as used herein, refers to all the different forms of the POLQ gene and may be produced by mutations, or may arise from the same gene by alternative splicing. A large number of isoforms are caused by single nucleotide polymorphisms or SNPs, small genetic differences between alleles of the same gene. These occur at specific individual nucleotide positions within a gene. Also included within this definition is the situation where different versions of messenger RNA are created from the same gene by employing different promoters, which causes transcription to skip certain exons. Thus, it is understood that the methods of the invention are not restricted to POLQ per se, but also encompass one or several POLQ isoforms. According to methods of the invention, the level of the expression of the POLQ gene and/or one or several of its isoforms is measured, and ratios of expression are calculated.

According to the present invention, "aggressiveness" of a cancer is intended to mean the propensity of said cancer to invade the neighboring tissues and to generate metastases and the rapidity with which said invasions may appear. Aggressiveness of the cancer is obviously correlated to survival, and the above method may be used for prognosing survival of the patient, in which case diagnosing of aggressiveness results in a bad survival prognosis and diagnosis of the absence of aggressiveness results in a good survival prognosis.

As used herein, "genetic instability" of a cancer is intended to mean the propensity of tumor cells of said cancer to suffer from DNA damage or "replication stress" (such as reactivation of dormant origins). Genetic instability is the hallmark of cancer in which DNA damage are more frequent. By "DNA damage", it is herein meant injury to DNA that affects the normal function of the DNA by causing covalent modification of the DNA, DNA breaks, or causing it to deviate from its normal double-helical conformation. DNA damage includes structural distortions which interfere with replication and transcription, as well as point mutations which can disrupt base pairs and can change the DNA sequence. In general, when a cell incurs DNA damage, the cell cycle is arrested at one of three checkpoints (GI/S, intra-S or G2/M). The cell cycle arrest can lead to the activation of DNA repair processes (in the case of relatively minor DNA damage), or result in the induction of apoptosis (in the case of catastrophic DNA damage).

DNA damage can be caused spontaneously by endogenous processes such as oxidation of bases and generation of DNA strand interruptions by reactive oxygen and free radicals produced from normal metabolism, methylation of bases, depurination, depyrimidination, mismatch of bases by DNA polymerases during DNA replication, etc. DNA damage can also be caused by environmental insults such as radiation (e.g., ultraviolet radiation, x-rays, gamma rays, ionizing radiation), natural toxins (e.g., plant toxins), synthetic toxins, drugs (e.g., cancer chemotherapy, radiation therapy), alkylating agents, etc. DNA damage can lead to or result from a variety of disorders, including hereditary genetic disorders and disorders as a result of exposure to environmental insults. DNA damage can also occur as a result of smoking (smoke including genotoxic aromatic compounds), leading to for example, heart disease , or as a result of therapies for other diseases, such as cancers (including lung cancer).

The above methods are performed using a cancer sample of the patient to be tested. In some cases, the methods according to the disclosure may further comprise a preliminary step of taking a cancer sample from the patient. By a "cancer sample", it is referred to a tumor tissue sample. Even in a cancerous patient, the tissue which is the site of the tumor still comprises non tumor healthy tissue. The "cancer sample" should thus be limited to tumor tissue taken from the patient. Said "cancer sample" may be a biopsy sample or a sample taken from a surgical resection therapy.

In addition, the methods according to the disclosure may comprise another preliminary step, between the taking of the sample from the patient and steps a) as defined above, corresponding to the transformation of the cancer sample (and optionally of the healthy tissue sample) into a mRNA (or corresponding cDNA) sample or into a protein sample, which is then ready to use for in vitro measuring of genes expression levels in step a). Preparation or extraction of mRNA (as well as retrotranscription into cDNA) or proteins from a tissue sample is only routine procedure well known to those skilled in the art.

Once a ready-to-use cancer mRNA (or corresponding cDNA) or protein sample is available, the measure of POLQ gene expression levels may be performed, depending on the type of transformation and the available ready-to-use sample, either at the mRNA (i.e. based on the mRNA content of the sample) or at the protein level (i.e. based on the protein content of the sample). In some embodiments, the expression levels of some of the genes may be measured at the mRNA level, while the expression levels of other genes are measured at the protein level. In this case, part of the cancer sample taken from the patient has been transformed into an mRNA (or corresponding cDNA) sample and another part has been transformed into a protein sample. In other embodiments, the expression levels of all tested genes are measured either at the mRNA or at the protein level.

When expression levels are measured at the mRNA level, it may be notably performed using well known technologies such as quantitative PCR or nucleic acid microarray technologies (including cDNA and oligonucleotide microarrays). These technologies are now used routinely by those skilled in the art and thus do not need to be detailed here. Examples of embodiments using quantitative PCR are described in the experimental section. Alternatively, any known or future technology permitting to assess genes expression levels based on mRNA contents may be used. For instance, tissue microarrays coupled to fluorescent in situ hybridization may be used. Tissue microarrays (also known as TMAs) consist of paraffin blocks in which up to 1000 separate tissue cores are assembled in array fashion to allow multiplex histological analysis. In the tissue microarray technique, a hollow needle is used to remove tissue cores as small as 0.6 mm in diameter from regions of interest in paraffin-embedded tissues such as clinical biopsies or tumor samples. These tissue cores are then inserted in a recipient paraffin block in a precisely spaced, array pattern. Sections from this block are cut using a microtome, mounted on a microscope slide and then analyzed by any method of standard histological analysis. Each microarray block can be cut into 100 - 500 sections, which can be subjected to independent tests. Tests commonly employed in tissue microarray include immunohistochemistry, and fluorescent in situ hybridization. For analysis at the mRNA level, tissue microarray technology may be coupled to fluorescent in situ hybridization.

When expression levels are measured at the protein level, it may be notably performed using specific antibodies, in particular using well known technologies such as western blot, ELISA or ELISPOT, antibodies microarrays, or tissue microarrays coupled to immunohistochemistry.

The comparison of the expression levels of the measured genes in said patient's cancer sample is made by calculating an expression level ratio of the expression level of the POLQ gene to the expression level of a control gene in said patient's cancer sample, and by comparing the obtained expression level ratio to a corresponding threshold value. Said control gene, according to the present invention, is a gene which is expressed in all cell types. More specifically, the control gene according to the invention is a gene which is expressed in all the cells constituting the tissue which is the site of the tumor. In another aspect, the expression level of the control gene is not affected by the state of the cell, i.e. the control gene is expressed to the same level in a healthy cell and in a tumor cell. In a specific embodiment, the control gene is a housekeeping gene. A housekeeping gene is a gene expressed in all cell types, which provides a basic function needed for sustenance of all cell types. A list of human housekeeping genes may be found in Eisenberg et al. (Trends in Genetics 19: 362-365, 2003). A preferred housekeeping gene according to the invention is a gene selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. A further preferred housekeeping gene according to the invention is selected from the group consisting of IPO8, HMBS, GUSB, and UBC.

According to the present invention, a "threshold value" is intended to mean a value that permits to discriminate samples in which the expression level ratio of the gene of interest corresponds to an expression level of said gene of interest in the patient's cancer sample that is low or high. In particular, if a gene expression level ratio is inferior or equal to the threshold value, then the expression level of this gene in the patient's cancer sample is considered low, whereas if a gene expression level ratio is superior to the threshold value, then the expression level of this gene in the patient's cancer sample is considered high. For each gene, and depending on the method used for measuring the expression level of the genes, the optimal threshold value may vary. However, it may be easily determined by a skilled artisan based on the analysis of several control cancer samples in which the expression level (low or high) is known for this particular gene, and on the comparison thereof with the expression of a control gene, e.g. a housekeeping gene.

The present disclosure further relates to a microarray dedicated to the implementation of the methods according to the invention, comprising at most 500, preferably at most 300, at most 200, more preferably at most 150, at most 100, even more preferably at most 75, at most 50, at most 40, at most 30, at most 20, at most 10 distinct probes, at least 1 of which specifically binds to POLQ mRNA (or corresponding cDNA) or protein. In a preferred embodiment, said microarray is a nucleic acid microarray, comprising at most 500, preferably at most 300, at most 200, more preferably at most 150, at most 100, even more preferably at most 75, at most 50, at most 40, at most 30, at most 20, at most 10 distinct probes (thus excluding for instance pangenomic microarrays), at least 1 of which specifically hybridizes to POLQ mRNA (or corresponding cDNA). Said microarray may also contain at least one probe which specifically hybridizes to a housekeeping gene in addition to the probe specifically hybridizing to POLQ. In one embodiment, said housekeeping gene is selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. More preferentially, the housekeeping gene is selected from the group consisting of the IPO8, HMBS, GUSB, and UBC genes. According to the disclosure, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray", the oligonucleotides being about 25 to about 60 base pairs or less in length).

Alternatively, in another embodiment, said microarray may be an antibodies microarray, comprising at most 500, preferably at most 300, at most 200, more preferably at most 150, at most 100, even more preferably at most 75, at most 50, at most 40, at most 30, at most 20, at most 10 distinct antibodies, at least 1 of which specifically bind to POLQ protein. Said microarray may also contain at least one antibody which specifically binds to a housekeeping protein, in addition to the antibody specifically binding to the POLQ protein. In one embodiment, said housekeeping protein is selected in the group consisting of the B2M, TFRC, YWHAZ, RPLO, 18S, GUSB, UBC, TBP, GAPDH, PPIA, POLR2A, ACTB, PGK1, HPRT1, IPO8 and HMBS proteins. In a preferred embodiment, said housekeeping protein is selected from the group consisting of the IPO8, HMBS, GUSB, and UBC proteins.

Alternatively to nucleic acid or antibody microarray technology, quantitative PCR may be used and amplification primers specific for the genes to be tested are thus also very useful for performing the methods according to the invention. The present disclosure thus further relates to a kit for diagnosing aggressiveness and genetic instability of a cancer in a patient from a cancer sample of said patient, comprising a dedicated microarray as described above or amplification primers specific for POLQ. Here also, when the kit comprises amplification primers, while said kit may comprise amplification primers specific for other genes, said kit preferably comprises at most 100, at most 75, 50, at most 40, at most 30, preferably at most 25, at most 20, at most 15, more preferably at most 10, at most 8, at most 6, even more preferably at most 5, at most 4, at most 3 or even 2 or one or even zero couples of amplification primers specific for other genes than POLQ. For example, said kit may comprise at least a couple of amplification primers for at least one housekeeping gene in addition to the primers for POLQ. In one embodiment, said housekeeping gene is selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS. In a preferred embodiment, said housekeeping gene is selected from the group consisting of the IPO8, HMBS, GUSB, and UBC genes.

As mentioned above, the ability of prognosing cancer evolution, which is linked to its aggressiveness, is very important for selecting a suitable treatment, since heavy and costly treatments with potentially severe adverse effects should be used, in addition to traditional surgical treatment, each time they are necessary, but only when they are necessary. The present invention thus also concerns a method for choosing a suitable treatment of cancer in a patient, comprising:
a) diagnosing or not aggressiveness of said cancer in said patient using the method according to claim 1, and
b) adding adjuvant radiotherapy or chemotherapy to surgical treatment if said cancer is diagnosed as aggressive in step a), wherein the said cancer is adenocarcinoma of the lung.

In addition to its prognostic value concerning aggressiveness of cancer, the inventors also found that the same test based on the analysis of the expression levels of POLQ also allows diagnosing the presence or absence of genetic instability, thus resulting in the above described method for diagnosing genetic instability. In particular, the present inventors have shown that deregulated expression of POLQ is associated with an increased frequency of DNA breaks. In one preferred embodiment, the method of the disclosure is thus used to diagnose DNA breaks.

Such genetic instability, and notably the increased frequency of DNA breaks, may have consequences concerning the selection of an adjuvant therapy. In particular, while genetic instability may favor tumor cells mutations and thus deregulation of proliferation and adhesion, the presence of DNA damage might also be used against tumor cells. Indeed, for continued proliferation, those DNA breaks have to be repaired, and cells with a high number of DNA damage are usually prone to cell death. As a result, while radiotherapy may not be efficient in all circumstances, its efficiency on tumor cells that already present an increased frequency of DNA breaks may be improved. For example, radiotherapy may be highly efficient against the POLQ-overexpressing tumor cells identified by the method of the invention, because these cells contain high levels of DNA breakage and chromosomal instability. In the same manner, the use of DNA repair inhibitors might permit to freeze DNA breaks and lead to tumor cells death.

Indeed, the inventors have shown that the inhibition of DNA repair leads to a decreased viability of POLQ-overexpressing cells. As shown in the experimental examples, POLQ-overexpressing cells show increased sensitivity to DNA repair inhibitors.

The present invention thus also concerns a method for prognosing the efficiency of radiotherapy or DNA repair inhibitors in the treatment of cancer in a patient, comprising:
a) diagnosing or not genetic instability in said cancer in said patient using the method according to claim 2, and
b) prognosing efficiency of radiotherapy or DNA repair inhibitors if genetic instability has been diagnosed in step a), wherein the said cancer is adenocarcinoma of the lung.

According to the invention, a "DNA repair inhibitor" is intended to mean a molecule that is able to inhibit repair of DNA breaks, in particular double stranded DNA breaks. While this expression should not be understood as limitative, examples of DNA repair inhibitors include inhibitors of DNA repair protein PARP (see e.g. WO 2004080976, WO 2005/053662, WO 2009/046205), inhibitors of histone deacetylase, such as those described in PCT application WO 2008/082856, and inhibitors of DNA polymerase β (see WO2007001684). A "DNA damage checkpoint inhibitor" is a molecule which is capable of blocking the activity of any of the proteins involved in the DNA damage checkpoint. Examples of such proteins include ATM/ATR, Chk2 and Chk1. A "DNA replication licensing/initiation inhibitor".is a molecule capable of blocking the activity of any of the proteins involved in DNA replication licensing, such as Cdt1, Cdc6, Mcm1-7, and other known to the skilled person.

The present disclosure also relates to a method for treating a patient suffering from a cancer overexpressing POLQ, comprising subjecting said patient to radiotherapy and/or administering to said patient an effective amount of one ore more DNA repair inhibitors.

### FIGURES LEGENDS

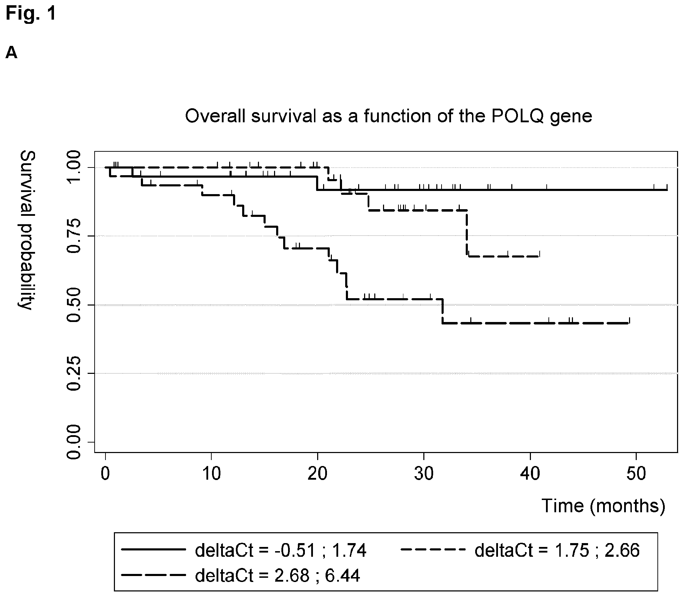

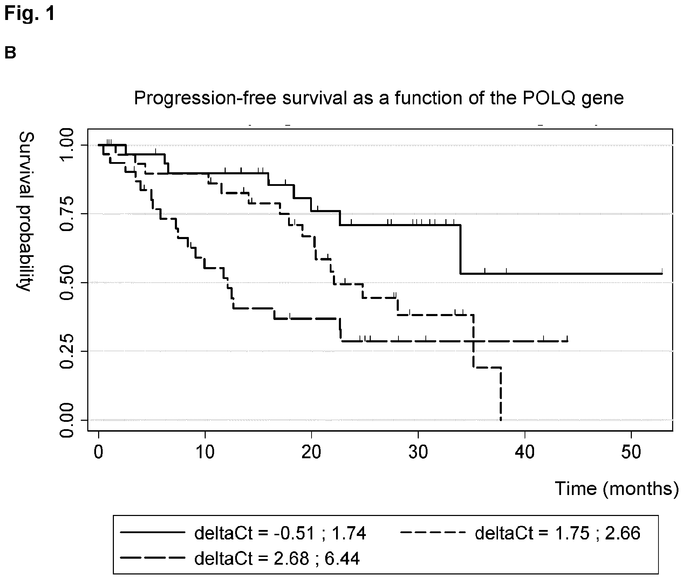

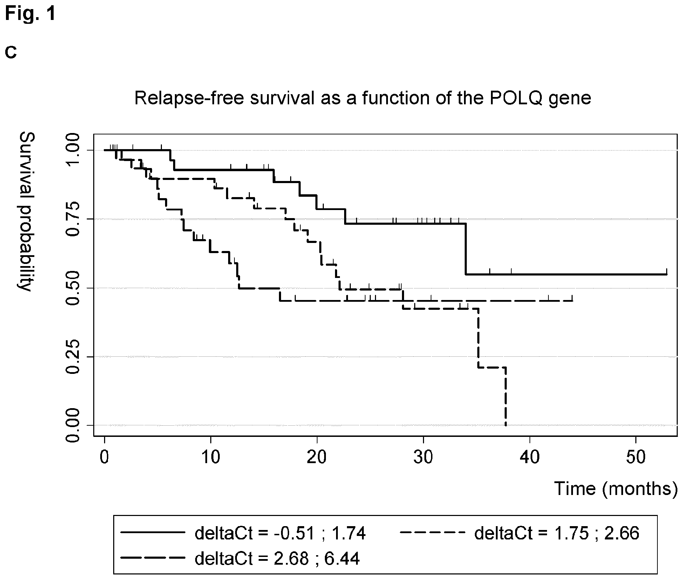

**Figure 1****. Effect of *POLQ* gene expression level on cancer-specific survival of patients.** (A) Kaplan-Meier overall survival of pulmonary adenocarcinoma patients, according to level of DNA *POLQ* expression in the primary tumor compared to adjacent normal tissue. Patients: n=93; p values taken from each log-rank test are indicated. (B) Kaplan-Meier progression-free survival of pulmonary adenocarcinoma patients, according to level of DNA *POLQ* expression in the primary tumor compared to adjacent normal tissue. Patients: n=93; p values taken from each log-rank test are indicated. (C) Kaplan-Meier relapse-free survival of pulmonary adenocarcinoma patients, according to level of DNA *POLQ* expression in the primary tumor compared to adjacent normal tissue. Patients: n=93; p values taken from each log-rank test are indicated.

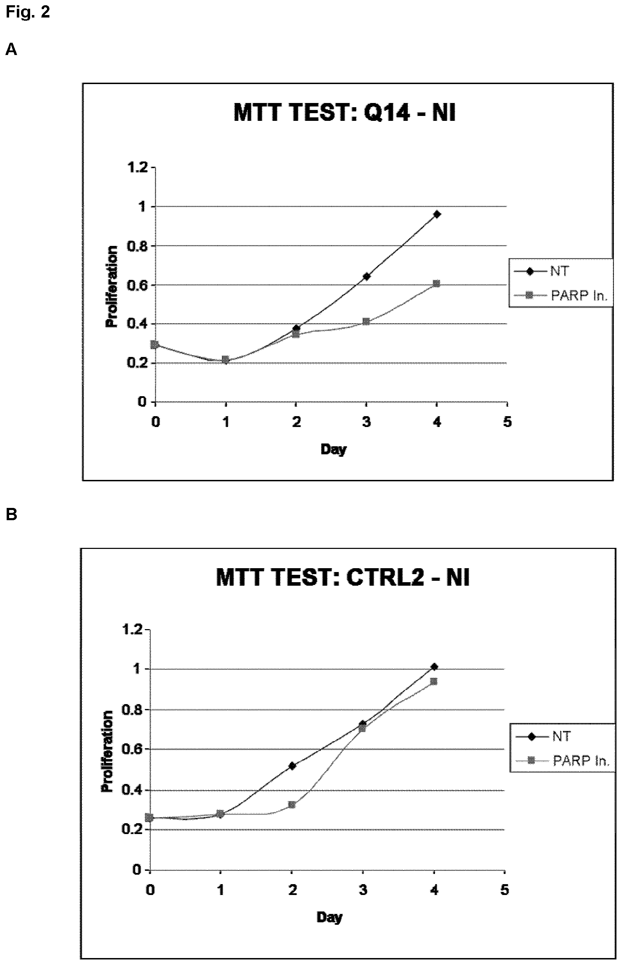

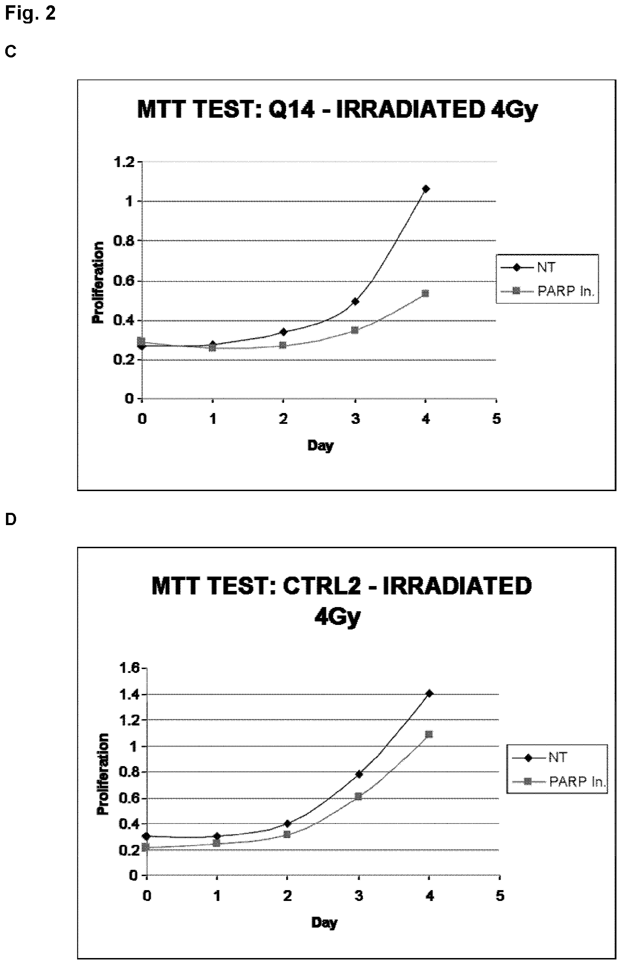

**Figure 2****. Effect of POLQ gene overexpression on cell sensitivity to DNA repair inhibitors** Cultures of lung MRC5 fibroblasts transfected with a a pcDNA POLQ-carrying vector (A) or with the corresponding naked vector (C) were treated with a sub-efficient dose of a PARP inhibitor, and cellular proliferation was monitored. Cultures of MRC5 cells transfected with a POLQ-carrying vector (B) or with the corresponding naked vector (D) were irradiated at sub-lethal doses (4 Gy), and cellular proliferation was monitored in the presence of PARP inhibitor.

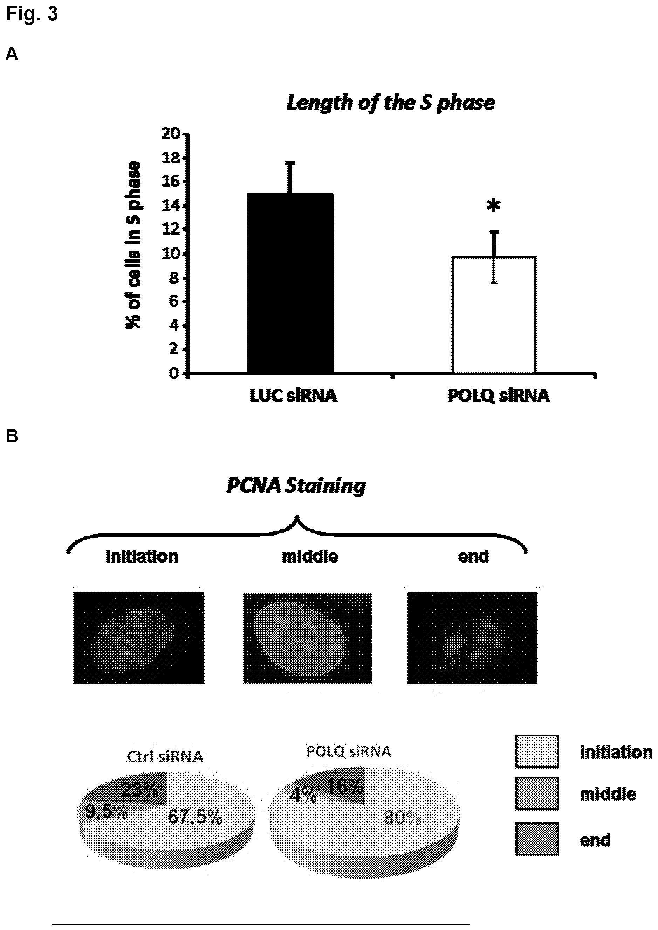

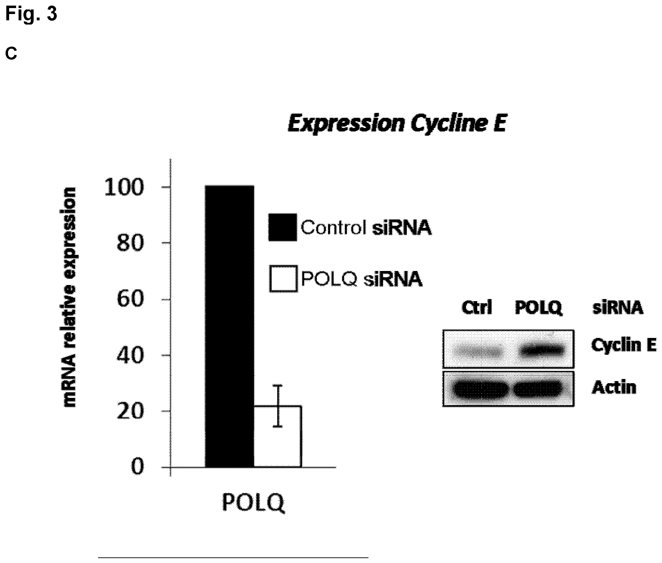

**Figure 3****. Effect of POLQ depletion on DNA replication** The effect of POLQ depletion on S phase was assessed in MRC5 cells transfected with POLQ siRNA, and compared to a LUC siRNA control (Ctrl): (A) Quantification of the average number of cells in S phase 8 hours after a thymidine chase; (B) Quantification of the average number of cells positive to PCNA staining; (C) Quantification of the cyclin E mRNA levels relative to the actin transcripts.

### EXAMPLES

### 1. Material and methods

### 1.1. Study design, patients and tumor samples, differential gene expression

Ninety-three patients carrying a pulmonary adenocarcinoma of stage **I** to IIIA were selected at the Centre Hospitalier Universitaire Rangueil-Larrey of Toulouse (France) between 2006 and 2010. Criteria assessed for inclusion of patients in the study were the availability of frozen tumoral and adjacent, non-tumoral tissues, and the possibility of obtaining RNA samples of a very high quality. Exclusion criteria were non-adenocarcinomous histology, a tumor of stage IIIB or IV, or a percentage of tumor cells of less than 70 %.

The tumor stage was determined on the basis of clinical and anatomopathological data using the TNM system. Analysis of the tumor morphology by the anatomopathologists was performed according to the World Health Organization classification of 2004 (Travis, William D; Brambilla, Elisabeth; Muller-Hermelink, H Konrad et al., eds, Pathology and Genetics of Tumours of the Lung, Pleura, Thymus and Heart, World Health Organization Classification of Tumours, Lyon: IARC Press, 2004). Only the tumors containing above 70 % of tumoral cells were retained for the present analysis.

All the tumor tissue samples were surgically collected and immediately snap-frozen in liquid nitrogen and stored until RNA extraction. Tumor RNA was prepared using the RNeasy Mini Kit (Qiagen) according to the manufacturer instructions. During the extraction process, all tumor samples were controlled for sufficient tumoral cellularity (i.e. >70% tumor cells) by performing haematoxylin-eosin staining on sixty 10 µm-thick or five 300 µm-thick sections of the frozen tissue. Likewise, slides for each normal sample were stained with haematoxylin-eosin slides and analyzed by a pathologist. Each normal sample was reviewed in order to (i) ascertain the absence of any neoplastic lesion; (ii) quantify the percentage of normal epithelial, fibrous and adipous components. The quality and the quantity of all French RNA samples were assessed using the Agilent BioAnalyzer 2100 and only RNA displaying a suitable ratio 28S/18S (≥ 1.7) and a suitable RNA integrity number (RIN≥ 7) was selected for analysis.

Total RNA was reverse transcribed using the High-Capacity cDNA Archive Kit (Applied Biosystems). The level of transcripts was measured in triplicates by using the TaqMan low density array (Applied Biosystems). All studied genes were amplified in triplicate from tumor and normal samples using the TaqMan Universal PCR Master Mix and the TaqMan Low Density Array technology (Applied Biosystems). PCR amplifications were performed with the TaqMan Low Density Array technology and either the 7900HT fast real time PCR system. Gene expression was normalized between samples by using four controls (*GUSB, HMBS, IPO8,* and *UBC*), which were amplified from each cDNA. These four control genes were selected by using the GeNorm program as the most stable among 16 tested (*B2M, TFRC, YWHAZ, RPLO,* 18S, *GUSB, UBC, TBP, GAPDH, PPIA, POLR2A, ACTB, PGK1, HPRT1, IPO8, HMBS*) on the TaqMan Low Density Human Endogenous Control Array (Applied Biosystems).

The primer DNA context sequences as well as the Applied Biosystems references used for analysis of each tested and control genes were selected on the Applied Biosystem web site (http://products.appliedbiosystems;com). The cDNA was amplified in presence of the different probes diluted 100-fold and of the TaqMan® Preamp Master Mix (Early Access) reagent (Applied Biosystems) for 14 cycles in the StepOne real-time PCR system (Applied Biosystems). Pre-amplification products were then diluted 5-fold. Pre-amplified cDNA were then resuspended in DNA Binding Sample Loading Reagent buffer (Applied Biosystems) in presence of Master Mix (Applied Biosystems). Meanwhile, each probe is diluted to 1/100 in Assay Loading Reagent buffer (Applied Biosystems). Amplification is performed on a Fluidigm Biomark™. The preamplified cDNAs and the probes are separately loaded onto 96.96 Dynamic Array (Fluidigm). Detection of real-time PCR reaction is performed on the Biomark Reader (Fluidigm).

Parameter of analysis were as suggested by the manufacturer (quality threshold = 0.65; baseline correction = linear; Ct threshold method = auto global). Fluidigm PCR data were generated as monoplicates; they were then analyzed with the GenEx software (http://genex.gene-quantification.info/, MultiD), with the following protocol: &) normalization of the data with respect to the household genes identified by TLDA (GUSB, IPO8, UBC, HMBS): Ct in the tissue - mean of the Ct of the 4 household genes in the same tissue, 2) normalization of tumor tissue with respect to adjacent non-tumor tissue: number of RNA copies = 2^Ct. Tumor tissue can be compared to the adjacent non-tumor tissue with the ratio: Nrel = 2^CtNormal/2^CtTumor = 2^(CtNormal - CtTumor). A gene is overexpressed if Nrel > 1, a gene is underexpressed if Nrel < 1, 3/ binomial transformation: in order to obtain a normal distribution allowing the performance of statistical tests: Log2(Nrel) = Log2(2^CtNormal/2^CtTumor) = Ct Normal - Ct Tumor. A gene is overexpressed if CtHealthy - CtTumor > 0, a gene is underexpressed if CtHealthy - CtTumoral < 0.

### 1.2. Statistical Analysis

As a first step, for each of the studied genes, the probability of observing more than 50 % of the patients overexpressing or underexpressing the said gene (threshold : Nrel > 2 for overexpression and Nres < 1/2 for underexpression) was assessed by a binomial test. The significance level was 0.05 and the procedure of Benjamini and Yekutieli was used to assess for the multiplicity of tests. Correlations between genes were assessed with a non parametric Spearman's correlation (Spearman's rho). Expression levels (classified in 3 categories according to the terciles of the Nrel distribution) were compared in relation to the treatment (surgery only, surgery-chemotherapy-radiotherapy, or surgery-chemotherapy), to the tumor grade (N or TNM), of the tumor differentiation (poorly-, moderately-, or well-differentiated), of the presence of emboli, and of the smoking habits by chi-square or Fisher's exact test. Survival of the patients in relation to the expression levels (in 3 categories) was evaluated by the Kaplan Meier method and compared by log-Rank test. Survival in relation to the expression levels of the genes was analysed in multivariate with a Cox model.

### 1.3. Subcloning of POLQ

The human *POLQ* cDNA was transferred into the vector pcDNA 3.1 (Invitrogen) in two steps. First the pFast Bac HTC POLQ containing *POLQ* cDNA (Seki et al. Nucleic Acids Res. 31:6117-6126, 2003) was digested with *RsrII* and *XhoI* to give the N-terminal part of *POLQ* cDNA (1.2 kb). This product was subsequently inserted into pcDNA 3.1 (Invitrogen), previously digested with *EcoRV* and *XhoI.* The C-terminal part of *POLQ* cDNA (6.7 kb) was then isolated by digestion of the pFast Bac HTC POLQ with *XhoI* and *SacII* and inserted in the pcDNA 3.1 vector already containing the N-terminal part of POLQ cDNA after digestion of this vector with *XhoI.* The entire sequence of the *POLQ* cDNA was then confirmed.

### 1.4. Cell engineering

MRC5-SV cells (ATCC) were grown and transfected as described (Pillaire et al. Cell Cycle 6: 471-7, 2007). Cell cycle analysis studies were performed as also published (Bergoglio et al. J Cell Science 115: 4413-4418, 2002). Cell cycle analysis and cytotoxicity studies were performed as previously published (Bergoglio et al. J Cell Science 115: 4413-4418, 2002). Cells were collected 3 hours after colcemid treatment and metaphase spreads were prepared. For each clone, a minimum of 100 metaphases were analyzed in 3 independent experiments; p-values were calculated by Student's t-test (Bergoglio et al. Cancer Res 62: 3511-3514, 2002).

### 2. Results

### 2.1. Most of DNA replication genes are deregulated in coupled lung tumours.

Gene expression profiles of 93 coupled primary lung adenocarcinomas at different stages of progression (Table 1) were generated from a selection of more than 80 genes known as playing a role in initiation/licensing of DNA replication, translesional (TLS) or conventional DNA elongation, DNA damage response (DDR), DNA fork protection or repair of replication-induced double-stranded breaks.

In particular, POLQ was shown by this real-time PCR analysis to be highly expressed in most of the 93 patients. Indeed, POLQ is up-regulated in tumor tissues (T) compared to adjacent control tissues (N).of the said patients (Table 1).

**Table 1: Differential expression of POLQ s in coupled NSCLC tumours**

| **Replication gene** | **DNA transaction** | **N° coupled tumors** | **Over-expression (ΔCt>1)** | **Underexpression (ΔCt<1)** | **P value** |
|---|---|---|---|---|---|
| PolQ | TLA DNA replication | 93 | 75 | 19 | 0.00000004 |

As shown in Table 2, we observed that the POLQ gene was more than 2-fold (T/N >2) over-regulated.

**Table 2: exact binomial tests (P values <0.05)**

| Replication gene | DNA transaction | T/N overexpression ratio | | | |
|---|---|---|---|---|---|
| | | >5 | >4 | >3 | >2 |
| POLQ | TLS DNA replication | | | P<6 10⁻⁵ | P<1 10⁻¹⁰ |

### 2.2. Deregulated 3R expression is associated with poor prognosis

A log-rank test for equality of survivor functions indicated that up-regulation of the POLQ (p<0.0033) DNA polymerase gene was associated with disease-free survival. When relapse-free survival of patients was investigated, overexpression of this gene was again significantly related to the outcome of patients (p<0.05). Finally we analyzed the disease-free survival criteria and found that POLQ (p<0.0008) was associated with a higher morbidity when overall survival was measured (Fig. 1).

**Table 3: Multivariate analysis - cancer specific survival (n = 93)**

| **Gene Terciles** | **Log rank Test** | | | **Khi2 or Fisher's exact Tests** | |
|---|---|---|---|---|---|
| | pvalue≤0.05 → significant effect of gene expression on survival (OS, PFS, TTP) | | | pvalue≤0.05 → significant association between treatment or stade N and gene expression | |
| | pvalue>0.05 → non significant effect of gene expression on survival (OS, PFS, TTP) | | | | |
| | | | | pvalue>0.05 → non significant association between treatment or stade N and gene expression. | |
| | **Overall survival (OS)** | **Progression -free survival (PFS)** | **Relapse-free survival (RFS)** | **Treatment** | **Stade N** |
| | | | | -Untreated patients (n=46) | -N0 (n=61 |
| | | | | | |
| | Evt=death | | | | -N1 (n=11) |
| | | Evt=death or relapse | Evt=relapse | -Patients treated by chemo + radio (n=11) | -N2+N3 (n=21) |
| | | | | - Patients treated by chemo only (n=36) | |
| PolQ [-0.508,1.75] | **P=0,0008***** | **P=0.0033**** | **P=0.0359*** | 0.348 | 0.7193 |

| | | | | | |
|---|---|---|---|---|---|
| * *significance* with p < 0,05 ** : significance with p < 0,01 *** : significance with p < 0,001 | | | | | |

Overexpression of POLQ is therefore associated with survival whatever the criteria used to assess survival. Importantly, this association is independent of the tumor stage and of the treatment.

### 2.3. Overexpression of POLQ leads to genetic abnormalities

To investigate the impact of POLQ over-expression on genomic stability, MRC5 cells were transfected with either a POLQ-carrying plasmid or the corresponding empty plasmid. Metaphase spreads from the transfected clones (Q1, Q2, Q3) and isogenic controls were analysed. POLQ overexpression resulted in a significant increase in the frequency of cells displaying quadriradials, end-to-end fusions and chromatid breaks (25% of aberrant metaphases were detected in POLQ cells versus 13% in controls; Table 4). These data indicate that spontaneous chromosome instability occurs in POLQ over-expressing cells, which may be the consequences of increased DNA breakage.

**Table 4: Chromosomal aberrations in POLQ-overexpressing MRC5 cells**

| Chromosomal aberrations | CTL2 | CTL9 | Q1 | Q2 | Q3 |
|---|---|---|---|---|---|
| N° of Metaphases | 292 | 290 | 305 | 320 | 301 |
| N° of chromatid breaks | 36 | 28 | 72 | 85 | 57 |
| N° of end-to-end fusions | 12 | 10 | 32 | 24 | 32 |
| N° of dicentric chromosomes | 16 | 10 | 34 | 17 | 15 |
| Other abnormalities | 3 | 7 | 9 | 14 | 7 |
| Total N° of abnormalities | 67 | 55 | 147 | 140 | 111 |
| | | | p=0.03 | p=0.003 | p=0.009 |
| % Cells with abnormalities | 10.5 | 13.8 | 24.6 | 26.6 | 25.6 |
| N° of abnormalities per cell | 0.23 | 0.19 | 0.48 | 0.44 | 0.37 |

### 2.4. POLQ-overexpressing cells show higher sensitivity to DNA-repair inhibitors

A proposed role of POLQ in Base Excision Repair (BER) or alternative Non Homologous End Joining (NHEJ) could be that overexpressed POLQ interferes with the BER and/or B-NHEJ pathways and leads to an accumulation of endogenous DNA damage (including damage generated by reactive oxygen species).

To test this hypothesis, POLQ-overexpressing MRC5 cells (Q14) were either treated with a sub-efficient dose of a PARP inhibitor or left untreated. As shown in Figure 2, the proliferation of MRC5 cells overexpressing POLQ was significantly affected when these cells were treated with the said PARP inhibitor, consistent with a delayed repair of the DNA damages. Likewise, Q14 cells showed significant delay in cell proliferation after irradiation at a sub-lethal dose. By contrast, MRC5 cells which did not overexpress POLQ were not affected by either treatment with the PARP inhibitor or mild irradiation.

### 2.5. POLQ biological role is linked to entry into S phase

The role of POLQ in replication was investigated by transfecting POLQ-specific siRNA into MRC5 cells and monitoring the cell-cycle status of POLQ-depleted cells. It was immediately clear that the number of cells in S phase was lower in POLQ siRNA-expressing cells than in control cells, indicating a perturbation of the S-phase progression. This interpretation was confirmed by immunostaining of the PCNA protein, a marker of S cells, in both control and transfected cells: a significant increase in cells displaying a staining consistent with the beginning of the DNA replication was observed in the POLQ siRNA transfected cell population, but not in the control.

This perturbation of S phase could be due to an effect on replication initiation, on replication elongation, or on both. In order to assess the effect of the inhibition POLQ in DNA replication initiation, cyclin E mRNA (*CCNE1,* Genbank ID: NM_001238; and *CCNE2,* Genbank ID: NM_057749) was evaluated by Northern blotting in POLQ-depleted cells as well as in control cells. When normalized to actin transcript levels, cyclin E transcript levels were clearly more elevated in POLQ siRNA-expressing cells. Since cyclin E is required for the onset of S phase, POLQ depletion leads to an increased replication initiation activity. It is already known that POLQ affects fork velocities (Lemée et al., Proc Natl Acad Sci U S A., 107(30): 13390-5, 2010). Deregulation of POLQ expression thus interferes with the licensing for replication, triggering a replicative stress and the genetic instability in the tumors of the study.

## Claims

1. A method for diagnosing aggressiveness of a cancer in a patient from a cancer sample of said patient, comprising:
a) measuring in vitro the expression level of the POLQ gene and/or one or several of its isoforms, and the expression level of a control gene in said patient cancer sample,
b) calculating for said POLQ gene and/or isoform an expression level ratio of the expression level of POLQ and/or one or several of its isoforms to the expression of the said control gene in said patient cancer sample
c) comparing said gene expression level ratio to a corresponding threshold value, and
d) diagnosing cancer aggressiveness if the said ratio is superior to its corresponding threshold value,
wherein said cancer is adenocarcinoma of the lung.

2. A method for diagnosing genetic instability in a cancer in a patient from a cancer sample of said patient, comprising:
a) measuring in vitro the expression level of the POLQ gene and/or one or several of its isoforms, and the expression level of a control gene in said patient cancer sample
b) calculating for said POLQ gene and/or isoform an expression level ratio of the expression level of POLQ and/or one or several of its isoforms to the expression of the said control gene in said patient cancer sample
c) comparing said gene expression level ratio to a corresponding threshold value, and
d) diagnosing cancer genetic instability if the said ratio is superior to its corresponding threshold value,
wherein said cancer is adenocarcinoma of the lung.

3. The method of any one of claims 1 to 2, wherein said control gene is a housekeeping gene.

4. The method of claim 3, wherein said housekeeping gene is a gene selected in the group consisting of B2M, TFRC, YWHAZ, RPLO, 18S, GUSB, UBC, TBP, GAPDH, PPIA, POLR2A, ACTB, PGK1, HPRT1, IPO8 and HMBS.

5. The method of claim 4, wherein said gene is selected from the group consisting of IPO8, HMBS, GUSB, and UBC.

6. The method of any one of claims 1-5, wherein said expression level is measured at the mRNA level.

7. The method of claim 6, wherein said expression level is measured using quantitative PCR or microarray technology.

8. The method of any one of claims 1-7, wherein said expression level is measured at the protein level.

9. The method of claim 8, wherein said expression level is measured using specific antibodies.

10. A method for choosing a suitable treatment of cancer in a patient, comprising:
a) diagnosing or not aggressiveness of said cancer in said patient using the method of claim 1, and
b) adding adjuvant radiotherapy or chemotherapy to surgical treatment if said cancer is diagnosed as aggressive in step a), wherein the said cancer is adenocarcinoma of the lung.

11. A method for prognosing the efficiency of radiotherapy or DNA repair, DNA damage signaling or nucleotide metabolism inhibitors in the treatment of cancer in a patient, comprising:
a) diagnosing or not genetic instability in said cancer in said patient using the method of claim 2, and
b) prognosing efficiency of radiotherapy or DNA repair inhibitors if genetic instability has been diagnosed in step a), wherein the said cancer is adenocarcinoma of the lung.

## Patentansprüche

1. Verfahren zur Diagnose der Aggressivität eines Krebses bei einem Patienten aus einer Krebsprobe des Patienten, umfassend:
a) *in* vitro-Messen des Expressionsniveaus des POLQ-Gens und/oder einer oder mehrerer seiner Isoformen und des Expressionsniveaus eines Kontrollgens in der Krebsprobe des Patienten,
b) Berechnen, für das POLQ-Gen und/oder die Isoform, eines Expressionsniveau-Verhältnisses des Expressionsniveaus von POLQ und/oder einer oder mehrerer seiner Isoformen zur Expression des Kontrollgens in der Krebsprobe des Patienten
c) Vergleichen des Genexpressionsniveau-Verhältnisses mit einem entsprechenden Schwellenwert, und
d) Diagnostizieren der Aggressivität des Krebses, wenn das Verhältnis größer als sein entsprechender Schwellenwert ist,
wobei der Krebs ein Adenokarzinom der Lunge ist.

2. Verfahren zur Diagnose der genetischen Instabilität eines Krebses bei einem Patienten aus einer Krebsprobe des Patienten, umfassend:
a) *in vitro*-Messen des Expressionsniveaus des POLQ-Gens und/oder einer oder mehrerer seiner Isoformen und des Expressionsniveaus eines Kontrollgens in der Krebsprobe des Patienten
b) Berechnen, für das POLQ-Gen und/oder die Isoform, eines Expressionsniveau-Verhältnisses des Expressionsniveaus von POLQ und/oder einer oder mehrerer seiner Isoformen zur Expression des Kontrollgens in der Krebsprobe des Patienten
c) Vergleichen des Genexpressionsniveau-Verhältnisses mit einem entsprechenden Schwellenwert, und
d) Diagnostizieren der genetischen Instabilität des Krebses, wenn das Verhältnis größer als sein entsprechender Schwellenwert ist,
wobei der Krebs ein Adenokarzinom der Lunge ist.

3. Verfahren nach einem der Ansprüche 1 bis 2, wobei das Kontrollgen ein Haushaltsgen ist.

4. Verfahren nach Anspruch 3, wobei das Haushaltsgen ein Gen ist, ausgewählt aus der Gruppe, bestehend aus B2M, TFRC, YWHAZ, RPLO, 18S, GUSB, UBC, TBP, GAPDH, PPIA, POLR2A, ACTB, PGK1, HPRT1, IPO8 und HMBS.

5. Verfahren nach Anspruch 4, wobei das Gen ausgewählt ist aus der Gruppe, bestehend aus IPO8, HMBS, GUSB und UBC.

6. Verfahren nach einem der Ansprüche 1 - 5, wobei das Expressionsniveau auf dem mRNA-Niveau gemessen wird.

7. Verfahren nach Anspruch 6, wobei das Expressionsniveau unter Verwendung einer quantitativen PCR- oder Microarray-Technologie gemessen wird.

8. Verfahren nach einem der Ansprüche 1 - 7, wobei das Expressionsniveau auf dem Proteinniveau gemessen wird.

9. Verfahren nach Anspruch 8, wobei das Expressionsniveau unter Verwendung von spezifischen Antikörpern gemessen wird.

10. Verfahren zur Auswahl einer geeigneten Behandlung von Krebs bei einem Patienten, umfassend:
a) Diagnostizieren oder nicht der Aggressivität des Krebses bei dem Patienten unter Verwendung des Verfahrens nach Anspruch 1, und
b) Hinzufügen einer adjuvanten Strahlentherapie oder Chemotherapie zur chirurgischen Behandlung, wenn der Krebs in Schritt a) als aggressiv diagnostiziert wird, wobei der Krebs ein Adenokarzinom der Lunge ist.

11. Verfahren zur Prognose der Effizienz von Strahlentherapie oder DNA-Reparatur, DNA-Schadensignalisierung oder Nukleotidmetabolismus-Inhibitoren bei der Behandlung von Krebs bei einem Patienten, umfassend:
a) Diagnostizieren oder nicht der genetischen Instabilität des Krebses bei dem Patienten unter Verwendung des Verfahrens nach Anspruch 2, und
b) Prognostizieren der Effizienz von Strahlentherapie oder DNA-Reparatur-Inhibitoren, wenn die genetische Instabilität in Schritt a) diagnostiziert wurde, wobei der Krebs ein Adenokarzinom der Lunge ist.

## Revendications

1. Méthode de diagnostic de l'agressivité d'un cancer chez un patient à partir d'un échantillon cancéreux dudit patient, comprenant :
a) la mesure in vitro du niveau d'expression du gène POLQ et/ou d'une ou plusieurs de ses isoformes, et du niveau d'expression d'un gène témoin dans ledit échantillon cancéreux du patient,
b) le calcul pour ledit gène POLQ et/ou son isoforme d'un rapport de niveau d'expression du niveau d'expression de POLQ et/ou d'une ou plusieurs de ses isoformes sur l'expression dudit gène témoin dans ledit échantillon cancéreux du patient
c) la comparaison dudit rapport de niveau d'expression du gène à une valeur seuil correspondante, et
d) le diagnostic de l'agressivité du cancer si ledit rapport est supérieur à sa valeur seuil correspondante,
dans laquelle ledit cancer est un adénocarcinome pulmonaire.

2. Méthode de diagnostic de l'instabilité génétique dans un cancer chez un patient à partir d'un échantillon cancéreux dudit patient, comprenant :
a) la mesure in vitro du niveau d'expression du gène POLQ et/ou d'une ou plusieurs de ses isoformes, et du niveau d'expression d'un gène témoin dans ledit échantillon cancéreux du patient
b) le calcul pour ledit gène POLQ et/ou son isoforme d'un rapport de niveau d'expression du niveau d'expression de POLQ et/ou d'une ou plusieurs de ses isoformes sur l'expression dudit gène témoin dans ledit échantillon cancéreux du patient
c) la comparaison dudit rapport de niveau d'expression du gène à une valeur seuil correspondante, et
d) le diagnostic de l'instabilité génétique du cancer si ledit rapport est supérieur à sa valeur seuil correspondante,
dans laquelle ledit cancer est un adénocarcinome pulmonaire.

3. Méthode selon l'une quelconque des revendications 1 à 2, dans laquelle ledit gène témoin est un gène domestique.

4. Méthode selon la revendication 3, dans laquelle ledit gène domestique est un gène sélectionné dans le groupe constitué de B2M, TFRC, YWHAZ, RPLO, 18S, GUSB, UBC, TBP, GAPDH, PPIA, POLR2A, ACTB, PGK1, HPRT1, IP08 et HMBS.

5. Méthode selon la revendication 4, dans laquelle ledit gène est sélectionné dans le groupe constitué d'IPO8, HMBS, GUSB, et UBC.

6. Méthode selon l'une quelconque des revendications 1 - 5, dans laquelle ledit niveau d'expression est mesuré au niveau de l'ARNm.

7. Méthode selon la revendication 6, dans laquelle ledit niveau d'expression est mesuré en utilisant la technologie de PCR quantitative ou des micropuces.

8. Méthode selon l'une quelconque des revendications 1 - 7, dans laquelle ledit niveau d'expression est mesuré au niveau des protéines.

9. Méthode selon la revendication 8, dans laquelle ledit niveau d'expression est mesuré en utilisant des anticorps spécifiques.

10. Méthode de sélection d'un traitement approprié du cancer chez un patient, comprenant :
a) le diagnostic ou non de l'agressivité dudit cancer chez ledit patient en utilisant la méthode selon la revendication 1, et
b) l'ajout de radiothérapie ou chimiothérapie adjuvantes au traitement chirurgical si ledit cancer est diagnostiqué agressif à l'étape a), dans laquelle ledit cancer est un adénocarcinome pulmonaire.

11. Méthode de pronostic de l'efficacité de la radiothérapie ou des inhibiteurs de la réparation de l'ADN, de la signalisation des lésions de l'ADN ou du métabolisme des nucléotides dans le traitement du cancer chez un patient, comprenant :
a) le diagnostic ou non de l'instabilité génétique dans ledit cancer chez ledit patient en utilisant la méthode selon la revendication 2, et
b) le pronostic de l'efficacité de la radiothérapie ou des inhibiteurs de la réparation de l'ADN si l'instabilité génétique a été diagnostiquée à l'étape a), dans laquelle ledit cancer est un adénocarcinome pulmonaire.
